# EUROPÄISCHE PATENTSCHRIFT

(11) **EP 3 692 975 B1**
(45) Veröffentlichungstag und Bekanntmachung des Hinweises auf die Patenterteilung: **18.05.2022**
(21) Anmeldenummer: 19156083.8
(22) Anmeldetag: 07.02.2019
(51) Int. Cl.: A61K 6/887, A61K 6/61

(54) **DENTALMATERIALIEN MIT VERBESSERTEM ABBINDEVERHALTEN**
DENTAL MATERIALS WITH IMPROVED SETTING
MATIÈRES DENTAIRES AYANT UN COMPORTEMENT AMÉLIORÉ FACE AU DURCISSEMENT

(43) Veröffentlichungstag der Anmeldung: 12.08.2020
(73) Patentinhaber: Ivoclar Vivadent AG, 9494 Schaan (LI)
(72) Erfinder: MOSZNER, Norbert, 9495 Triesen (LI); GIANASMIDIS, Alexandros, 9436 Balgach (CH); WESTPHAL, Michael, 6800 Feldkirch (AT); BROT, Andy, 9496 Balzers (LI); GRABENBAUER, Barbara, 9445 Rebenstein (CH)
(74) Vertreter: Uexküll & Stolberg

(56) Entgegenhaltungen:
- EP-A1- 1 754 465
- EP-A1- 2 198 824
- EP-A1- 2 233 544
- WO-A1-2007/016508
- CN-A- 103 834 308
- JP-A- 2014 152 106
- US-A1- 2003 134 933

## Beschreibung

Die vorliegende Erfindung betrifft radikalisch polymerisierbare Zusammensetzungen mit verbessertem Abbindeverhalten, die sich insbesondere als Dentalmaterialien eigenen, beispielsweise als Prothesenmaterialien, Zemente, Adhäsive und Komposite für direkte Füllungen. Die Zusammensetzungen enthalten ein Redoxsystem als Initiator für die radikalische Polymerisation, das ein Hydroperoxid, ein Thioharnstoffderivat und ein Peroxid umfasst.

Haupteinsatzgebiete von Polymeren im Dentalbereich sind die abnehmbare (z.B. Zähne und Prothesenbasismaterialien) und festsitzende Prothetik (z.B. Verblendmaterialen, Kronen oder Zemente), Füllungsmaterialien (z.B. direkte oder indirekte Füllungskomposite, Befestigungszemente oder Adhäsive) oder Hilfsmaterialien (z.B. Abformmaterialien). Die Polymeren werden gewöhnlich durch radikalische Polymerisation geeigneter Zusammensetzungen erhalten, die eine polymerisierbare organische Matrix enthalten, in der Regel eine Mischung von Monomeren, Initiator-Komponenten und Stabilisatoren.

Als Monomere werden meistens Methylmethacrylat (MMA) (Prothesenmaterialien), Mischungen von funktionalisierten Monomeren, wie z.B. 2-Hydroxyethylmethacrylat (HEMA), oder säuregruppenhaltigen Haftmonomeren, wie z.B. 10-Methacryloyloxydecyldihydrogenphosphat (MDP), mit Dimethacrylaten (Adhäsive) oder Mischungen eingesetzt, die ausschließlich Dimethacrylate enthalten (Kompositzemente und Füllungskomposite). Häufig verwendete Dimethacrylate sind 2,2-Bis[4-(2-hydroxy-3-methacryloyloxypropyl)phenyl]propan (Bis-GMA) und 1,6-Bis-[2-methacryloyloxy-ethoxycarbonylamino]-2,2,4-trimethylhexan (UDMA), die eine hohe Viskosität haben und zu Polymerisaten mit sehr guten mechanischen Eigenschaften führen. Als reaktive Verdünner finden vor allem Triethylenglycoldimethacrylat (TEGDMA), 1,10-Decandioldimethacrylat (D3MA) oder Bis(3-methacryloyloxymethyl)tricyclo-[5.2.1.02.6]decan (DCP) Anwendung.

Die Aushärtung von methacrylatbasierenden Dentalmaterialien erfolgt durch radikalische Polymerisation, wobei je nach Anwendungsgebiet radikalische Photoinitiatoren (Lichthärtung, direkte Füllungskomposite und Adhäsive), thermische Initiatoren (indirekte Komposite oder Prothesenmaterialien) oder Redox-Initiatorsysteme (Kompositzemente) eingesetzt werden und wobei auch die Kombination von Photoinitiatoren mit Redoxinitiatoren, z.B. bei Füllungen tiefer Kavitäten, bekannt ist.

Redoxsysteme kommen vor allem dann zum Einsatz, wenn z.B. bei Prothesenmaterialien wegen einer geringen Monomerreaktivität oder bei Befestigungszementen aufgrund unzureichender Durchstrahlung eine unvollständige Aushärtung zu befürchten ist.

Um eine ausreichende Lagerstabilität der Materialien zu gewährleisten, werden Werkstoffe auf der Basis von Redoxinitiatoren meist als sogenannte ZweiKomponenten-Systeme (2K-Systeme) eingesetzt, wobei das Oxydationsmittel (Peroxid- oder Hydroperoxid) und das Reduktionsmittel (Amine, Sulfinsäuren, Barbiturate, Thioharnstoff etc.) in zwei getrennte Komponenten eingearbeitet werden. Diese Komponenten werden kurz vor der Anwendung miteinander gemischt. Dabei müssen die beiden Komponenten so abgestimmt sein, dass ihre homogene Vermischung und Anwendung einfach möglich ist und dass eine für dentale Zwecke ausreichende Verarbeitungszeit erreicht wird. Unter der Verarbeitungszeit wird die Zeitspanne zwischen dem Vermischen der beiden Komponenten und der beginnenden Aushärtung des gemischten Materials verstanden. Diese sollte in einem Bereich von etwa 90 und 150 s liegen. Andererseits darf die Aushärtungszeit, d.h. der Zeitraum bis zur vollständigen Härtung der Materialen, nicht zu lang sein. Eine Aushärtungszeit von ca. 3 bis 5 min ist optimal.

Für dentale Kompositzemente wurden für lange Zeit vor allem Redoxinitiatorsysteme eingesetzt, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) beruhen. Mit DBPO/Amin-basierenden Redoxinitiatorsystemen lassen sich die Verarbeitungs- und Aushärtezeit in Kombination mit phenolischen Inhibitoren relativ gut einstellen. Ein Nachteil solcher DBPO/Amin-Systeme sind Verfärbungen, die durch eine langsame Oxidation der Amine verursacht werden. Außerdem wird die Radikalbildung bei DBPO/Amin-basierenden Redoxinitiatorsystemen durch Säuren und damit auch durch saure Monomeren beeinträchtigt, die regelmäßig zur Herstellung von Schmelz-Dentin-Adhäsiven eingesetzt werden. Die Aminkomponente wird durch eine Säure-Base-Reaktion protoniert und dadurch deaktiviert.

Die voranstehenden Nachteile können mit Hydroperoxid-Redoxinitiatorsystemen zum Teil überwunden werden, weil keine tertiären Amine als Reduktionsmittel erforderlich sind. Außerdem sind Hydroperoxide thermisch stabiler als Peroxide. Cumolhydroperoxid weist beispielsweise eine 10-Stunden-Halbwertstemperatur T_{1/2} von 158 °C auf, die 10-Stunden-Halbwertstemperatur T_{1/2} von DBPO beträgt nur 73 °C.

Die DE 26 35 595 C2 offenbart polymerisierbare Zahnfüllmassen, die als Initiatorsystem ein substituiertes Thioharnstoff-Reduktionsmittel in Kombination mit einem Hydroperoxid-Oxydationsmittel enthalten. Die Materialien sollen eine verbesserte Farbbeständigkeit, eine hervorragende Härtegeschwindigkeit und eine verbesserte Lagerfähigkeit aufweisen.

Die EP 1 693 046 B1 offenbart dentale Zemente und Stumpfaufbaumaterialien, die ein (2-Pyridyl)-2-thioharnstoffderivat in Kombination mit einem Hydroperoxid enthalten, in dem die Hydroperoxidgruppe an ein tertiäres Kohlenstoffatom gebunden ist.

Die WO 2007/016508 A1 offenbart eine polymerisierbare dentale Zusammensetzung, die als Initiatorsystem ein Thioharnstoffderivat in Kombination mit einem Hydroperoxid enthält. Die Zusammensetzung enthält keine Monomeren mit Säuregruppen.

Gemäß EP 1 754 465 B1 weist das Cumolhydroperoxid/Acetylthioharnstoff-System eine unbrauchbar langsame Aushärtungskinetik auf. Zur Überwindung dieses Problems wird die Zugabe von löslichen Kupferverbindungen vorgeschlagen. Kupferionen sind gefärbt und können die Farbe der Materialien beeinflussen.

Die US 7,275,932 B2 schlägt die Verwendung von Hydroperoxiden und Thioharnstoffderivaten in Kombination mit einer sauren Verbindung als Beschleuniger vor. Bevorzugte saure Verbindungen sind Acrylate und Methacrylate mit Säuregruppen wie z.B. Methacrylsäure.

Die EP 2 233 544 A1 und die EP 2 258 336 A1 offenbaren Dentalwerkstoffe, die ein Hydroperoxid und ein Thioharnstoffderivat in Kombination mit einer Vanadiumverbindung als Beschleuniger enthalten. Vanadiumverbindungen können eine grünliche Verfärbung der Werkstoffe bewirken.

Die US 6,815,470 B2 schlägt zur Vermeidung der mit organischen Peroxiden und tertiären Aminen verbundenen Nachteile die Verwendung eines Arylborats in Kombination mit einer sauren Verbindung und einem Peroxid als Initiatorsystem vor. Das Arylborat soll durch Reaktion mit der sauren Verbindung ein Arylboran bilden, das bei der Reaktion mit Sauerstoff polymerisierbare Radikale freisetzt. Als saure Verbindung können polymerisierbare Monomere verwendet werden, die Säuregruppen aufweisen.

Die CN 103 834 308 A offenbart geruchsarme, zweikomponentige Adhäsive auf Acrylatbasis, die als Polymerisationsinitiator ein Peroxid, als Beschleuniger ein Amin, ein Thioharnstoffderivat, ein Aldehydaminkondensat oder eine Mischung davon und als zusätzlichen Beschleuniger eine organische Übergangsmetallverbindung enthalten.

Die JP 2014 152106 A offenbart härtbare Dentalzusammensetzungen, die als Polymerisationsinitiator eine Kombination eines Hydroperoxids mit einer Thioharnstoffverbindung und einer wasserlöslichen Kupferverbindung enthalten. Die Zusammensetzungen können zusätzlich Peroxide als weitere Polymerisationsinitiatoren enthalten.

Trotz der zahlreichen Bemühungen zur Überwindung der mit Peroxiden und Aminbeschleunigern verbundenen Nachteile ist bisher kein Initiatorsystem für dentale Zwecke gefunden worden, das in jeder Hinsicht befriedigend ist.

Der Erfindung liegt die Aufgabe zugrunde, Dentalmaterialien zur Verfügung zu stellen, die die Nachteile des Standes der Technik nicht aufweisen. Die Materialien sollen einerseits über eine hohe Lagerstabilität verfügen und keine Verfärbungen zeigen, gleichzeitig aber schnell härten und dennoch eine für dentale Zwecke geeignete Verarbeitungszeit aufweisen.

Diese Aufgabe wird durch aminfreie, radikalisch polymerisierbare Dentalwerkstoffe gelöst, die mindestens ein radikalisch polymerisierbares Monomer und als Initiatorsystem für die radikalische Polymerisation eine Kombination aus einem Thioharnstoffderivat und einem Hydroperoxid und zusätzlich mindestens ein Peroxid enthalten, wobei die Menge des Peroxids 1 bis 15 Gew.-% bezogen auf die Masse des Hydroperoxids beträgt. Erfindungsgemäß wurde überraschend gefunden, dass die Reaktivität eines Initiatorsystems auf der Basis eines Hydroperoxids und eines Thioharnstoffderivats durch den Zusatz einer geringer Mengen eines Peroxids deutlich beschleunigt werden kann.

Erfindungsgemäß bevorzugte Hydroperoxide sind Verbindungen der Formel R-(OOH)ₙ, in der R ein aliphatischer oder aromatischer Kohlenwasserstoffrest und n 1 oder 2 ist. Bevorzugte Reste R sind Alkyl- und Arylgruppen. Die Alkylgruppen können geradkettig, verzweigt oder cyclisch sein. Cyclische Alkylreste können durch aliphatische Alkylgruppen substituiert sein. Bevorzugt sind Alkylgruppen mit 4 bis 10 Kohlenstoffatomen. Arylgruppen können unsubstituiert oder durch Alkylgruppen substituiert sein. Bevorzugte aromatische Kohlenwasserstoffreste sind Benzolreste, die mit 1 oder 2 Alkylgruppen substituiert sind. Die aromatischen Kohlenwasserstoffreste enthalten vorzugsweise 6 bis 12 Kohlenstoffatome. Besonders bevorzugte Hydroperoxide sind t-Amylhydroperoxid, 1,1,3,3-Tetramethylbutylhydroperoxid, t-Butylhydroperoxid, t-Hexylhydroperoxid, 2,5-Dimethyl-2,5-di(hydroperoxy)hexan, Diisopropylbenzolmonohydroperoxid, Paramenthanhydroperoxid, p-Isopropylcumolhydroperoxid und Mischungen davon. Ganz besonders bevorzugt ist Cumolhydroperoxid (CHP).

Erfindungsgemäß bevorzugte Peroxide sind Verbindungen der Formel R'-(O-O-R")ₘ, in der R' und R" jeweils für einen aliphatischen oder aromatischen Kohlenwasserstoffrest oder eine Acylgruppe stehen und m 1 oder 2 ist. Besonders bevorzugt sind Diacylperoxide. Bevorzugte aliphatische Kohlenwasserstoffreste sind Reste mit 3 bis 8 Kohlenstoffatomen, bevorzugte aromatische Kohlenwasserstoffreste sind Reste mit 6 bis 12 Kohlenstoffatomen, wobei Benzolreste, die mit 1 oder 2 Alkylgruppen substituiert sind, besonders bevorzugt sind. Bevorzugte Acylgruppen sind Gruppen, die 2 bis 20 Kohlenstoffatome enthalten.

Bevorzugte Peroxide in denen R' und R" jeweils für einen aliphatischen oder aromatischen Kohlenwasserstoffrest stehen sind α,α-Bis(t-butylperoxy)diisopropylbenzol, Dicumolperoxid, 2,5-Dimethyl-2,5-bis(t-butylperoxy)hexan, t-Butylcumylperoxid, di-t-Butylperoxid, 2,5-Dimethyl-2,5-bis(t-butylperoxy)hexin-3.

Bevorzugte Diacylperoxide sind Isobutyrylperoxid, 2,4-Dichlorbenzoylperoxid, 3,5,5-Trimethylhexanoylperoxid, Octanoylperoxid, Lauroylperoxid, Stearylperoxid, Bernsteinsäureperoxid, m-Toluoylbenzoylperoxid und Mischungen davon. Ein ganz besonders bevorzugtes Peroxid ist Benzoylperoxid (DBPO). Hydroperoxide sind keine Peroxide im Sinne der Erfindung.

Bevorzugte Thioharnstoffderivate sind die in der EP 1 754 465 A1 in Absatz [0009] aufgeführten Verbindungen. Besonders bevorzugte Thioharnstoffderivate sind Acetyl-, Allyl-, Pyridyl- und Phenylthioharnstoff, Hexanoylthioharnstoff und Mischungen davon. Ganz besonders bevorzugt ist Acetylthioharnstoff (ATU).

Weiter bevorzugt sind Thioharnstoffderivate mit der Formel in der 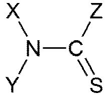
X H oder Y ist,
Y ein Alkylrest mit 1 bis 8 Kohlenstoffatomen, ein Cycloalkylrest mit 5 oder 6 Kohlenstoffatomen, ein Chlor-, Hydroxy- oder Mercapto-substituierter Alkylrest mit 1 bis 8 Kohlenstoffatomen, ein Alkenylrest mit 3 bis 4 Kohlenstoffatomen, ein Arylrest mit 6 bis 8 Kohlenstoffatomen, ein Chlor-, Hydroxy-, Methoxy- oder Sulfonyl-substituierter Phenylrest, ein Acylrest mit 2 bis 8 Kohlenstoffatomen, ein Chlor- oder Methoxy-substituierter Acylrest, ein Aralkylrest mit 7 bis 8 Kohlenstoffatomen oder ein Chlor- oder Methoxy-substituierter Aralkylrest ist, und
Z NH₂, NHX oder NX₂ ist.

Das Hydroperoxid wird vorzugsweise in einer Menge von 0,01 bis 5,0 Gew.-%, besonders bevorzugt 0,05 bis 4,0 Gew.-% und ganz besonders bevorzugt 0,1 bis 3,0 Gew.-% eingesetzt. Das Thioharnstoffderivat wird vorzugsweise in einer molaren Menge von 25 bis 100 mol-%, vorzugsweise 50 bis 100 mol-%, bezogen auf die molare Menge an Hydroperoxid, ganz besonders bevorzugt in der gleichen molaren Konzentration wie das Hydroperoxid eingesetzt. Das Peroxid wird in einer Menge von 1 bis 15 Gew.-%, bevorzugt 1 bis 10 Gew.-% und ganz besonders bevorzugt von 2 bis 8 Gew.-% eingesetzt, bezogen auf die Masse des Hydroperoxids.

Das erfindungsgemäße Initiatorsystem eignet sich besonders zur Aushärtung radikalisch polymerisierbarer Zusammensetzungen.

Erfindungsgemäße Zusammensetzungen enthalten neben dem Initiatorsystem mindestens ein radikalisch polymerisierbares Monomer. Bevorzugt sind Zusammensetzungen, die als radikalisch polymerisierbares Monomer mindestens ein mono- oder multifunktionelles (Meth)acrylat enthalten. Unter monofunktionellen (Meth)acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Gemäß einer besonders bevorzugten Ausführungsform enthalten die erfindungsgemäßen Zusammensetzungen mindestens ein Dimethacrylat oder eine Mischung von Mono- und Dimethacrylaten. Materialien, die intraoral gehärtet werden sollen, enthalten als radikalisch polymerisierbares Monomer vorzugsweise mono- und/oder multifunktionelle Methacrylate.

Bevorzugte mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumylphenoxyethylenglycolmethacrylat (CMP-1E), 2-(2-Biphenyloxy)-ethylmethacrylat, Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, wie z.B. 2-[4-(2-Methacryloyloxyethoxyethoxy)phenyl]-2-[4-(2-methacryloyloxyethoxy)phenyl]propan) (SR-348c, Firma Sartomer; enthält 3 Ethoxygruppen) und 2,2-Bis[4-(2-methacryloxypropoxy)phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tricyclo-[5.2.1.02,6]decan (DCP), Polyethylenglycol- oder Polypropylenglycoldimethacrylate, wie z.B. Polyethylenglycol-200- oder Polyethylenglycol-400-dimethacrylat (PEG-200- oder PEG-400-DMA) oder 1,12-Dodecandioldimethacrylat, oder eine Mischung davon.

Gemäß einer Ausführungsform enthalten die erfindungsgemäßen Zusammensetzungen neben den oben genannten Monomeren vorzugsweise zusätzlich ein oder mehrere säuregruppenhaltige radikalisch polymerisierbare Monomere (Haftmonomere). Diese verleihen den Werkstoffen selbsthaftende und/oder selbstätzende Eigenschaften. Säuregruppenhaltige Monomere eignen sich daher besonders zur Herstellung von selbsthaftenden Dentalwerkstoffen, wie z.B. von Befestigungszementen.

Bevorzugte säuregruppenhaltige Monomere sind polymerisationsfähige Carbonsäuren, Phosphonsäuren und Phosphorsäureester sowie deren Anhydride. Bevorzugte Carbonsäuren und Carbonsäureanhydride sind 4-(Meth)acryloyloxyethyltrimellit-säureanhydrid, 10-Methacryloyloxydecylmalonsäure, N-(2-Hydroxy-3-methacryloyl-oxypropyl)-N-phenylglycin, 4-Vinylbenzoesäure. Bevorzugte Phosphorsäureester sind 2-Methacryloyloxyethyl-phenyl-hydrogenphosphat, 10-Methacryloyloxydecyldihydrogenphosphat (MDP) und Dipentaerythritpentamethacryloyloxyphosphat. Bevorzugte Phosphonsäuren sind 4-Vinylbenzylphosphonsäure, 2-[4-(Dihydroxy-phosphoryl)-2-oxa-butyl]-acrylsäure und deren Amide, Ester, wie z.B 2-[4-(Dihydroxy-phosphoryl)-2-oxa-butyl]-acrylsäure-2,4,6-trimethylphenylester.

Besonders bevorzugte säuregruppenhaltige Monomere sind 4-Vinylbenzylphosphonsäure, 2-[4-(Dihydroxyphosphoryl)-2-oxa-butyl]-acrylsäure und deren Amide, Ester, wie z.B 2-[4-(Dihydroxyphosphoryl)-2-oxa-butyl]-acrylsäure-2,4,6-trimethylphenyl-ester, (Meth)acrylamiddihydrogenphosphate, wie z.B. 6-Methacrylamidohexyl- oder 1,3-Bis(methacrylamido)-propan-2-yl-dihydrogenphosphat, und Mischungen davon. Diese besonders bevorzugten säuregruppenhaltigen Monomere zeichnen sich durch eine hohe Hydrolysestabilität aus.

Die erfindungsgemäßen Zusammensetzungen können neben dem erfindungsgemäßen Initiatorsystem vorteilhaft zusätzlich einen Initiator für die radikalische Photopolymerisation enthalten. Solche Zusammensetzungen sind dualhärtend, d.h. sie lassen sich sowohl chemisch als auch durch Licht härten. Bevorzugte Photoinitiatoren sind Benzophenon, Benzoin sowie deren Derivate, α-Diketone und deren Derivate, wie 9,10-Phenanthrenchinon, 1-Phenyl-propan-1,2-dion, Diacetyl und 4,4'-Dichlorbenzil.

Die erfindungsgemäßen Zusammensetzungen enthalten keine Amine. Besonders bevorzugt sind daher Norrish-Typ-I-Photoinitiatoren. Norrish-Typ-I-Photoinitiatoren benötigen keine Aminkomponente.

Bevorzugte Norrish-Typ-I-Photoinitiatoren sind Acyl- oder Bisacylphosphinoxide. Besonders bevorzugt sind Monoacyltrialkylgermanium-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman und Tetrakis(o-methylbenzoyl)german.

Außerdem lassen sich auch Mischungen der verschiedenen Photoinitiatoren einsetzen.

Die erfindungsgemäßen Zusammensetzungen können vorteilhaft außerdem einen oder mehrere organische oder anorganische Füllstoffe enthalten. Bevorzugt sind partikuläre Füllstoffe. Füllstoffhaltige Zusammensetzungen eignen sich besonders als dentale Befestigungszemente oder Füllungskomposite.

Bevorzugte anorganische Füllstoffe sind Oxide, wie SiO₂, ZrO₂ und TiO₂ oder Mischoxide aus SiO₂, ZrO₂, ZnO und/oder TiO₂, nanopartikuläre oder mikrofeine Füllstoffe, wie pyrogene Kieselsäure oder Fällungskieselsäure, Glaspulver, wie Quarz-, Glaskeramik-, Borosilikat- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid. Weiterhin können die erfindungsgemäßen Dentalwerkstoffe faserförmige Füllstoffe, Nanofasern, Whiskers oder Mischungen davon enthalten.

Bevorzugt weisen die Oxide eine Partikelgröße von 0,010 bis 15 µm, die nanopartikulären oder mikrofeinen Füllstoffe eine Partikelgröße von 10 bis 300 nm, die Glaspulver eine Partikelgröße von 0,01 bis 15 µm, vorzugweise von 0,2 bis 1,5 µm, und die röntgenopaken Füllstoffe eine Partikelgröße von 0,2 bis 5 µm auf.

Besonders bevorzugte Füllstoffe sind Mischoxide aus SiO₂ und ZrO₂, mit einer Partikelgröße von 10 bis 300 nm, Glaspulver mit einer Partikelgröße von 0,2 bis 1,5 µm, insbesondere röntgenopake Glaspulver von z.B. Barium- oder Strontiumaluminiumsilikatgläsern, und röntgenopake Füllstoffe mit einer Partikelgröße von 0,2 bis 5 µm, insbesondere Ytterbiumtrifluorid und/oder Mischoxide von SiO₂ mit Ytterbium(III)-oxid.

Außerdem sind als Füllstoff gemahlene Präpolymerisate oder Perlpolymerisate (Isofüller) geeignet. Diese können ausschließliche aus organischen Polymeren oder aus organischen Polymeren bestehen, die ihrerseits mit anorganischen Füllstoffen wie röntgenopakem/n Glaspulver(n) und Ytterbiumtrifluorid gefüllt sind. Zur Herstellung der gemahlenen Präpolymerisate und Perlpolymere eignen sich die oben definierten Monomere und Füllstoffe. Zusammensetzungen zur Herstellung dentaler Totalprothesen enthalten als Füllstoffe vorzugsweise ausschließlich organische Füllstoffe, besonders bevorzugt gemahlene Polymerisate oder Perlpolymerisate auf Basis von Polymethylmethacrylat (PMMA), ganz besonders bevorzugt Perlpolymerisate auf der Basis von PMMA.

Wenn nicht anders angegeben, handelt es sich bei allen Partikelgrößen um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Malvern Zetasizer Nano ZS (Malvern Instruments, Malvern UK) mit einem He-Ne-Laser mit einer Wellenlänge von 633 nm, bei einem Streuwinkel von 90° bei 25°C.

Partikelgrößen kleiner als 0,1 µm können auch mittels REM- oder TEM-Spektroskopie bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 A dickem Kupfergitter (Maschenweite 300) aufgebracht, das mit Kohlenstoff beschichtet ist, und im Anschluss das Lösungsmittel verdampft.

Die Lichtstreuung nimmt mit abnehmender Partikelgröße ab, jedoch haben Füllstoffe mit geringer Partikelgröße eine größere Verdickungswirkung. Die Füllstoffe werden nach ihrer Partikelgröße unterteilt in Makrofüller und Mikrofüller, wobei Füllstoffe mit einer mittleren Partikelgröße von 0,2 bis 10 µm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Mikrofüller wie Mischoxide können z.B. durch hydrolytische Co-Kondensation von Metallalkoxiden hergestellt werden.

Zur Verbesserung des Verbundes zwischen den Füllstoffpartikeln und der vernetzten Polymerisationsmatrix sind die Füllstoffe vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Zur Oberflächenmodifizierung von nicht-silikatischen Füllstoffen, z.B. von ZrO₂ oder TiO₂, können auch funktionalisierte saure Phosphate, wie z.B. 10-Methacryloyloxydecyldihydrogenphosphat eingesetzt werden.

Außerdem können die erfindungsgemäßen Zusammensetzungen ein oder mehrere weitere Additive enthalten, vor allem Stabilisatoren, Farbmittel, mikrobiozide Wirkstoffe, fluoridionenabgebende Additive, Treibmittel, optische Aufheller, Weichmacher und/oder UV-Absorber.

Erfindungsgemäß sind Zusammensetzungen bevorzugt, die
(a) 0,01 bis 5,0 Gew.-%, bevorzugt 0,05 bis 4,0 Gew.-% und besonders bevorzugt 0,1 bis 3,0 Gew.-% Hydroperoxid, vorzugsweise CHP,
(b) 0,001 bis 0,75 Gew.-%, bevorzugt 0,005 bis 0,60 Gew.-% und besonders bevorzugt 0,005 bis 0,45 Gew.-% Peroxid, vorzugsweise DBPO,
(c) 0,001 bis 5,0 Gew.-%, bevorzugt 0,003 bis 4,0 Gew.-%, besonders bevorzugt 0,005 bis 3,0 Gew.-% Thioharnstoffderivat,
(d) 5 bis 95 Gew.-%, bevorzugt 10 bis 95 Gew.-% und besonders bevorzugt 10 bis 90 Gew.-% radikalisch polymerisierbares Monomer,
(e) 0 bis 85 Gew.-% Füllstoff, und
(f) 0,01 bis 5 Gew.-%, bevorzugt 0,1 bis 3 Gew.-% und besonders bevorzugt 0,1 bis 2 Gew.-% Additiv enthalten.

Alle Mengenangaben hierin beziehen sich auf die Gesamtmasse der Zusammensetzung, wenn nicht anders angegeben.

Der Füllungsgrad richtet sich nach dem gewünschten Anwendungszweck des Werkstoffs. Füllungskomposite haben vorzugsweise einen Füllstoffgehalt von 50 bis 85 Gew.-%, besonders bevorzugt 70 bis 80 Gew.-%, und dentale Zemente von 10 bis 70 Gew.-%, besonders bevorzugt 60 bis 70 Gew.-%.

Besonders bevorzugt sind solche Zusammensetzungen, die aus den genannten Stoffen bestehen. Weiterhin sind solche Zusammensetzungen bevorzugt, bei denen die einzelnen Komponenten jeweils aus den oben genannten bevorzugten und besonders bevorzugten Stoffen ausgewählt sind. In allen Fällen kann jeweils eine einzelne Komponente oder eine Mischung von mehreren Komponenten eingesetzt werden, also beispielsweise eine Mischung von Monomeren.

Die erfindungsgemäßen Zusammensetzungen eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Die Zusammensetzungen eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch nicht-therapeutisch (extraoral) eingesetzt werden, beispielsweise bei der Herstellung oder Reparatur von Dentalrestaurationen, wie Prothesen, künstlichen Zähnen, Inlays, Onlays, Kronen und Brücken.

Die erfindungsgemäßen Zusammensetzungen eignen sich außerdem zur Herstellung von Formkörpern für dentale aber auch für nicht dentale Zwecke, die z.B. mittels Gießen, Pressen und insbesondere durch generative Verfahren wie den 3D Druck hergestellt werden können.

Die Erfindung wird im Folgenden anhand von Ausführungsbeispielen näher erläutert:

### Ausführungsbeispiele

### Beispiel 1

### Chemisch härtende Zemente auf der Basis von CHP, ATU und DBPO

Durch Mischen der Dimethacrylate UDMA (Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), D3MA (1,10-Decandioldimethacrylat), GDMA (Glycerin-1,3-dimethacrylat) und der monofunktionellen Monomeren CMP-1E (p-Cumyl-phenoxyethylenglycolmethacrylat) und MDP (10-Methacryloyloxydecyldihydrogenphosphat) sowie dem Stabilisator BHT (2,6-Di-tert.-butyl-4-methylphenol), den Initiatorkomponenten CHP (Cumolhydroperoxid, 80 %-ig), DBPO (Dibenzoylperoxid, 50 %-ig) und ATU (Acetylthioharnstoff) wurden die in Tabelle 1 angegebenen Basen-Paste Base-1 und die Katalysator-Pasten Kat-1 bis Kat-5 hergestellt.

**Tabelle 1: Zusammensetzung der Katalysatorpasten Kat-1 bis Kat-5 und der Basenpaste Base-1 (Angaben in Gew.-%)**

| **Komponente** | **Kat-1*⁾** | **Kat-2** | **Kat-3** | **Kat-4** | **Kat-5** | **Base-1** |
|---|---|---|---|---|---|---|
| UDMA | 21,15 | 21,15 | 21,15 | 21,15 | 21,15 | 24,60 |
| V-380 | 16,92 | 16,92 | 16,92 | 16,92 | 16,92 | 19,68 |
| GDMA | 16,92 | 16,92 | 16,92 | 16,92 | 16,92 | 19,68 |
| D3MA | 12,69 | 12,69 | 12,69 | 12,69 | 12,69 | 14,76 |
| CMP-1E | 16,92 | 16,92 | 16,92 | 16,92 | 16,92 | 19,68 |
| MDP | 11,00 | 11,00 | 11,00 | 11,00 | 11,00 | - |
| BHT | 0,10 | 0,10 | 0,10 | 0,10 | 0,10 | 0,10 |
| CHP (80 %-ig) | 4,30 | 4,218 | 4,131 | 4,046 | 3,873 | - |
| DBPO (50 %-ig) | - | 0,086 | 0,173 | 0,258 | 0,431 | - |
| ATU | - | - | - | - | - | 1,50 |

| | | | | | | |
|---|---|---|---|---|---|---|
| *⁾ Vergleichsbeispiel | | | | | | |

Die Katalysator-Pasten wurden jeweils im Volumenverhältnis 1:1 mit der Basen-Paste vermischt und die Verarbeitungs- und Aushärtungszeit der erhaltenen Zemente H-1 bis H-5 bestimmt. Die Bestimmung der Verarbeitungszeit (VZ) erfolgte nach der Norm EN ISO-4049 (Dentistry - Polymer-based filling, restorative and luting materials). Hierzu wurden die Pasten unmittelbar nach dem Mischen in das Proberöhrchen einer Exotherm-Einrichtung mit Thermoelement (Thermoelement Typ K (Thermocoax FKI 10/50NN); Fa. Hersteller: THERMOCONTROL GmbH, Dietikon/Schweiz) eingefüllt, wobei die Zeitmessung mit Mischbeginn startete. Der Beginn der Aushärtung ist mit einer Temperaturerhöhung verbunden, welche die Exotherm-Einrichtung mit einem Kurvenanstieg anzeigt. Der Zeitpunkt des Temperaturanstieges entspricht dem Beginn der Härtungsreaktion und damit das Ende der Verarbeitungszeit. Die Verarbeitungszeit ist die Zeitphase von Mischbeginn bis zum Aushärtungsbeginn. Der Temperaturverlauf der Härtungsreaktion zeigt ein Maximum. Die Zeit des Maximums entspricht der Aushärtungszeit. Die Ergebnisse sind in Tabelle 2 dargestellt.

**Tabelle 2: Verarbeitungszeit (VZ) und Aushärtezeit (AZ) der Zemente H-1 bis H-5 (Angaben in Sekunden)**

| | **H-1*⁾** | **H-2** | **H-3** | **H-4** | **H-5** |
|---|---|---|---|---|---|
| VZ | 143 | 102 | 72 | 42 | 29 |
| AZ | 235 | 178 | 155 | 108 | 79 |

| | | | | | |
|---|---|---|---|---|---|
| *⁾ Vergleichsbeispiel | | | | | |

Die Ergebnisse belegen, dass die Zugabe geringer Mengen an DBPO, d.h. von weniger als 100 ppm DBPO, zu einer deutlichen Beschleunigung der Aushärtung der Harze führt.

### Beispiel 2

### Chemisch härtende Kompositzemente auf der Basis von CHP, ATU und DBPO

Durch Mischen der in Beispiel 1 beschriebenen Katalysatorpasten Kat-1 und Kat-3 und der Basenpaste Base-1 mit den Füllstoffen YbF₃ (Ytterbiumfluorid) und Sphärosil SiO2-ZrO2 sil. (silanisiertes SiO₂-ZrO₂-Mischoxid, Transparent Materials) wurden die füllstoffhaltigen Katalysatorpasten Kat-6 und Kat-7 sowie die füllstoffhaltige Basenpaste Base-2 mit den in Tabelle 3 angegebenen Zusammensetzungen hergestellt.

**Tabelle 3: Zusammensetzung der Katalysatorpasten Kat-6, Kat-7 und der Basenpaste Base-2 (Angaben in Gew.-%)**

| **Komponente** | **Kat-6** | **Kat-7** | **Base-2** |
|---|---|---|---|
| UDMA | 7,93 | 7,93 | 9,23 |
| V-380 | 6,34 | 6,34 | 7,38 |
| GDMA | 6,34 | 6,34 | 7,38 |
| D3MA | 4,76 | 4,76 | 5,53 |
| CMP-1E | 6,35 | 6,35 | 7,38 |
| MDP | 4,13 | 4,13 | - |
| BHT | 0,04 | 0,04 | 0,04 |
| CHP (80 %-ig) | 4,30 | 1,559 | - |
| DBPO (50 %-ig) | - | 0,065 | - |
| ATU | - | - | 0,56 |
| YbF₃¹⁾ | 20 | 20 | 20 |
| Sphärosil²⁾ | 42,50 | 42,50 | 42,50 |

| | | | |
|---|---|---|---|
| ' mittlere Korngröße: 250 nm ²⁾ silanisiertes SiO₂-ZrO₂-Mischoxid (Transparent Materials), Primärkorngröße d₅₀ = 60-80 nm, mittlere Korngröße ≤ 6µm | | | |

Die beiden Katalysatorpasten wurden jeweils im Volumenverhältnis 1:1 mit der Basenpaste Base-2 vermischt und die Verarbeitungs- und Aushärtungszeit der erhaltenen Kompositzemente C-1 und C-2 mittels eines oszillierenden Rheometers bestimmt (Tabelle 4). Dabei wurde die Konsistenzänderung der Zemente während der Verarbeitung und Aushärtung kontinuierlich aufgezeichnet, wobei die Konsistenzänderung über den Induktionsstrom detektiert wurde. Für die Datenaufzeichnung wurde der Datalogger Agilent 34970A verwendet. Für die Messungen wurde das zu prüfende Material auf Raumtemperatur (23,0 ± 2,0°C) vortemperiert. Während der Messung wurde der untere Rheometer-Messkopf auf 28,7±0,2 °C temperiert, was eine reale Messtemperatur von ca. 28-30°C ergab. Diese Temperatur entspricht der Temperatur, die unter intraoralen Bedingungen z.B. beim Zementieren einer Dentalrestauration vorliegt. Die Datenaufzeichnung erfolge mit Mischbeginn.

**Tabelle 4 Verarbeitungszeit (VZ) und Aushärtezeit (AZ) der Kompositzemente C-1 und C-2 (Angaben in Sekunden)**

| | **C-1***⁾ | **C-2** |
|---|---|---|
| VZ | 135 | 98 |
| AZ | 177 | 130 |

| | | |
|---|---|---|
| *⁾ Vergleichsbeispiel | | |

Die Ergebnisse belegen, dass die Zugabe geringer Mengen an DBPO zu einer deutlichen Beschleunigung der Aushärtung des füllstoffhaltigen Kompositzements führt.

## Patentansprüche

1. Radikalisch polymerisierbarer Dentalwerkstoff, der mindestens ein radikalisch polymerisierbares Monomer und als Initiatorsystem für die radikalische Polymerisation eine Kombination aus einem Thioharnstoffderivat und einem Hydroperoxid enthält, und kein Amin enthält,
**dadurch gekennzeichnet, dass** er zusätzlich mindestens ein Peroxid in einer Menge von 1 bis 15 Gew.-% bezogen auf die Masse des Hydroperoxids enthält, wobei Hydroperoxide keine Peroxide im Sinnes des Anspruchs sind.

2. Dentalwerkstoff nach Anspruch 1, der als Hydroperoxid eine Verbindung der Formel R-(OOH)ₙ enthält, in der R ein aliphatischer oder aromatischer Kohlenwasserstoffrest und n 1 oder 2 ist.

3. Dentalwerkstoff nach Anspruch 2, der als Hydroperoxid t-Amylhydroperoxid, 1,1,3,3-Tetramethylbutylhydroperoxid, t-Butylhydroperoxid, t-Hexylhydroperoxid, 2,5-Dimethyl-2,5-di(hydroperoxy)hexan, Diisopropylbenzolmonohydroperoxid, Paramenthanhydroperoxid, p-Isopropylcumolhydroperoxid oder eine Mischung davon, vorzugsweise Cumolhydroperoxid (CHP) enthält.

4. Dentalwerkstoff nach einem der vorangehenden Ansprüche, der als Peroxid α,α-Bis(t-butylperoxy)diisopropylbenzol, Dicumolperoxid, 2,5-Dimethyl-2,5-bis(t-butylperoxy)hexan, t-Butylcumylperoxid, di-t-Butylperoxid, 2,5-Dimethyl-2,5-bis(t-butylperoxy)hexin-3 oder eine Mischung davon enthält.

5. Dentalwerkstoff nach einem der vorangehenden Ansprüche, der als Peroxid ein Diacylperxoxid enthält, vorzugsweise Isobutyrylperoxid, 2,4-Dichlorbenzoylperoxid, 3,5,5-Trimethylhexanoylperoxid, Octanoylperoxid, Lauroylperoxid, Stearylperoxid, Bernsteinsäureperoxid, m-Toluoylbenzoylperoxid oder eine Mischung davon, vorzugsweise Benzoylperoxid (DBPO).

6. Dentalwerkstoff nach einem der vorangehenden Ansprüche, der als Thioharnstoffderivat Acetyl-, Allyl-, Pyridyl-, Phenylthioharnstoff, Hexanoylthioharnstoff oder eine Mischung davon enthält, vorzugsweise Acetylthioharnstoff (ATU).

7. Dentalwerkstoff nach einem der vorangehenden Ansprüche, der
- 0,01 bis 5,0 Gew.-%, bevorzugt 0,05 bis 4,0 Gew.-% und besonders bevorzugt 0,1 bis 3,0 Gew.-% Hydroperoxid, bezogen auf die Gesamtmasse des Werkstoffs,
- 25 bis 100 mol-%, bevorzugt 50 bis 100 mol-% und besonders bevorzugt eine äquimolare Menge Thioharnstoffderivat, bezogen auf die molare Menge an Hydroperoxid,
- 1 bis 15 Gew.-%, bevorzugt 1 bis 10 Gew.-% und besonders bevorzugt 2 bis 8 Gew.-% Peroxid, bezogen auf die Masse des Hydroperoxids, enthält.

8. Dentalwerkstoff nach einem der vorangehenden Ansprüche, der als radikalisch polymerisierbares Monomer mindestens ein mono- oder multifunktionelles (Meth)acrylat enthält, bevorzugt mindestens ein Dimethacrylat oder eine Mischung von Mono- und Dimethacrylaten.

9. Dentalwerkstoff nach Anspruch 8, der als radikalisch polymerisierbares Monomer Methyl-, Ethyl-, 2-Hydroxyethyl-, Butyl-, Benzyl-, Tetrahydrofurfuryl-oder Isobornyl(meth)acrylat, p-Cumyl-phenoxyethylenglycolmethacrylat, 2-(2-Biphenyloxy)-ethylmethacrylat, Bisphenol-A-dimethacrylat, Bis-GMA, ethoxy-oder propoxyliertes Bisphenol-A-Dimethacrylat, 2-[4-(2-Methacryloyloxy-ethoxyethoxy)phenyl]-2-[4-(2-methacryloyloxyethoxy)phenyl]propan), 2,2-Bis[4-(2-methacryloxypropoxy)phenyl]propan, ein Additionsprodukt aus 2-Hydroxyethylmethacrylat und 2,2,4-Trimethylhexamethylen-1,6-diisocyanat, ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat, Di-, Tri- oder Tetraethylenglycoldimethacrylat, Trimethylolpropantrimethacrylat, Pentaerythrittetramethacrylat, Glycerindi- und -trimethacrylat, 1,4-Butandioldimethacrylat, 1,10-Decandioldimethacrylat, Bis(methacryloyloxymethyl)tricyclo-[5.2.1.02,6]decan, ein Polyethylenglycol-oder Polypropylenglycoldimethacrylat, Polyethylenglycol-200-, Polyethylenglycol-400-dimethacrylat, 1,12-Dodecandioldimethacrylat oder eine Mischung davon enthält.

10. Dentalwerkstoff nach Anspruch 8 oder 9, der zusätzlich mindestens ein säuregruppenhaltiges radikalisch polymerisierbares Monomer enthält, vorzugsweise eine polymerisationsfähige Carbonsäure, Phosphonsäure, einen polymerisationsfähigen Phosphorsäureester oder ein Anhydrid dieser Stoffe.

11. Dentalwerkstoff nach einem der vorangehenden Ansprüche, der zusätzlich mindestens einen organischen oder anorganischen Füllstoff enthält, vorzugsweise ein Oxid, wie SiO₂, ZrO₂ und TiO₂ oder ein Mischoxid aus SiO₂, ZrO₂, ZnO und/oder TiO₂, einen nanopartikulären oder mikrofeinen Füllstoff, wie pyrogene Kieselsäure oder Fällungskieselsäure, Glaspulver, wie Quarz-, Glaskeramik- oder röntgenopakes Glaspulver, vorzugsweise Barium- oder Strontiumaluminiumsilikatglaspulver, einen röntgenopaken Füllstoff, wie Ytterbiumtrifluorid, Tantal(V)-oxid, Bariumsulfat, ein Mischoxid von SiO₂ mit Ytterbium(III)-oxid oder Tantal(V)-oxid, ein gemahlenes Präpolymerisat oder ein Perlpolymerisat.

12. Dentalwerkstoff nach einem der vorangehenden Ansprüche, der
(a) 0,01 bis 5,0 Gew.-%, bevorzugt 0,05 bis 4,0 Gew.-% und besonders bevorzugt 0,1 bis 3,0 Gew.-% Hydroperoxid, vorzugsweise Cumolhydroperoxid,
(b) 0,001 bis 0,75 Gew.-%, bevorzugt 0,005 bis 0,60 Gew.-% und besonders bevorzugt 0,005 bis 0,45 Gew.-% Peroxid, vorzugsweise Benzoylperoxid,
(c) 0,001 bis 5,0 Gew.-%, bevorzugt 0,003 bis 4,0 Gew.-%, besonders bevorzugt 0,005 bis 3,0 Gew.-% Thioharnstoff-Derivat,
(d) 5 bis 95 Gew.-%, bevorzugt 10 bis 95 Gew.-% und besonders bevorzugt 10 bis 90 Gew.-% radikalisch polymerisierbares Monomer,
(e) 0 bis 85 Gew.-% Füllstoff, und
(f) 0,01 bis 5 Gew.-%, bevorzugt 0,1 bis 3 Gew.-% und besonders bevorzugt 0,1 bis 2 Gew.-% Additiv enthält,
jeweils bezogen auf die Gesamtmasse des Werkstoffs.

13. Dentalwerkstoff nach Anspruch 12, der 50 bis 85 Gew.-% oder 10 bis 70 Gew.-% Füllstoff enthält.

14. Dentalwerkstoff nach einem der vorangehenden Ansprüche zur therapeutischen Anwendung, vorzugsweise als dentaler Zement, Füllungskomposit oder Verblendmaterial.

15. Nicht-therapeutische Verwendung eines Dentalwerkstoffs gemäß einem der Ansprüche 1 bis 13 zur Herstellung oder Reparatur von Dentalrestaurationen, wie Prothesen, künstlichen Zähnen, Inlays, Onlays, Kronen, Brücken und Totalprothesen.

## Claims

1. Radically polymerizable dental material, which comprises at least one radically polymerizable monomer and a combination of a thiourea derivative and a hydroperoxide as initiator system for the radical polymerization and does not comprise an amine, **characterized in that** it additionally comprises at least one peroxide in a quantity of from 1 to 15 wt.-% relative to the mass of the hydroperoxide, wherein hydroperoxides are not peroxides within the meaning of this claim.

2. Dental material according to claim 1, which comprises a compound of the formula R-(OOH)ₙ, in which R is an aliphatic or aromatic hydrocarbon radical and n is 1 or 2, as hydroperoxide.

3. Dental material according to claim 2, which comprises t-amyl hydroperoxide, 1,1,3,3-tetramethylbutyl hydroperoxide, t-butyl hydroperoxide, t-hexyl hydroperoxide, 2,5-dimethyl-2,5-di(hydroperoxy)hexane, diisopropylbenzene mono-hydroperoxide, paramenthane hydroperoxide, p-isopropylcumene hydroperoxide or a mixture thereof, preferably cumene hydroperoxide (CHP), as hydroperoxide.

4. Dental material according to any one of the preceding claims, which comprises a,a-bis(t-butylperoxy)diisopropylbenzene, dicumene peroxide, 2,5-dimethyl-2,5-bis(t-butylperoxy)hexane, t-butyl cumyl peroxide, di-t-butyl peroxide, 2,5-dimethyl-2,5-bis(t-butylperoxy)hexyne-3 or a mixture thereof as peroxide.

5. Dental material according to any one of the preceding claims, which comprises a diacyl peroxide, preferably isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoyl benzoyl peroxide or a mixture thereof, preferably benzoyl peroxide (DBPO), as peroxide.

6. Dental material according to any one of the preceding claims, which comprises acetyl, allyl, pyridyl, phenyl thiourea, hexanoyl thiourea or a mixture thereof, preferably acetyl thiourea (ATU), as thiourea derivative.

7. Dental material according to any one of the preceding claims, which comprises
- 0.01 to 5.0 wt.-%, preferably 0.05 to 4.0 wt.-% and particularly preferably 0.1 to 3.0 wt.-% hydroperoxide, relative to the total mass of the material,
- 25 to 100 mol-%, preferably 50 to 100 mol-% and particularly preferably an equimolar quantity of thiourea derivative, relative to the molar quantity of hydroperoxide,
- 1 to 15 wt.-%, preferably 1 to 10 wt.-% and particularly preferably 2 to 8 wt.-% peroxide, relative to the mass of the hydroperoxide.

8. Dental material according to any one of the preceding claims, which comprises at least one mono- or multifunctional (meth)acrylate as radically polymerizable monomer, preferably at least one dimethacrylate or a mixture of mono- and dimethacrylates.

9. Dental material according to claim 8, which comprises methyl, ethyl, 2-hydroxyethyl, butyl, benzyl, tetrahydrofurfuryl or isobornyl (meth)acrylate, p-cumylphenoxyethylene glycol methacrylate, 2-(2-biphenyloxy)ethyl methacrylate, bisphenol A dimethacrylate, bis-GMA, ethoxylated or propoxylated bisphenol A dimethacrylate, 2-[4-(2-methacryloyloxyethoxyethoxy)phenyl]-2-[4-(2-methacryloyloxyethoxy)phenyl]propane), 2,2-bis[4-(2-methacryloxypro-poxy)phenyl]propane, an addition product of 2-hydroxyethyl methacrylate and 2,2,4-trimethylhexamethylene-1,6-diisocyanate, an addition product of a mixture of 0.7 mol 2-hydroxyethyl methacrylate and 0.3 mol 2-hydroxypropyl methacrylate with 1 mol α,α,α',α'-tetramethyl-m-xylylene diisocyanate, di-, tri-or tetraethylene glycol dimethacrylate, trimethylolpropane trimethacrylate, pentaerythritol tetramethacrylate, glycerol di- and trimethacrylate, 1,4-butanediol dimethacrylate, 1,10-decanediol dimethacrylate, bis(methacryloyloxymethyl)tri-cyclo-[5.2.1.02,6]decane, a polyethylene glycol or polypropylene glycol dimethacrylate, polyethylene glycol 200 dimethacrylate, polyethylene glycol 400 dimethacrylate, 1,12-dodecanediol dimethacrylate or a mixture thereof as radically polymerizable monomer.

10. Dental material according to claim 8 or 9, which additionally comprises at least one acid-group-containing radically polymerizable monomer, preferably a polymerizable carboxylic acid, phosphonic acid, a polymerizable phosphoric acid ester or an anhydride of these substances.

11. Dental material according to any one of the preceding claims, which additionally comprises at least one organic or inorganic filler, preferably an oxide, such as SiO₂, ZrO₂ and TiO₂ or a mixed oxide of SiO₂, ZrO₂, ZnO and/or TiO₂, a nanoparticulate or microfine filler, such as pyrogenic silica or precipitated silica, glass powder, such as quartz, glass ceramic or radiopaque glass powder, preferably barium or strontium aluminium silicate glass powder, a radiopaque filler, such as ytterbium trifluoride, tantalum(V) oxide, barium sulfate, a mixed oxide of SiO₂ with ytterbium(III) oxide or tantalum(V) oxide, a ground prepolymer or a pearl polymer.

12. Dental material according to any one of the preceding claims, which comprises
(a) 0.01 to 5.0 wt.-%, preferably 0.05 to 4.0 wt.-% and particularly preferably 0.1 to 3.0 wt.-% hydroperoxide, preferably cumene hydroperoxide,
(b) 0.001 to 0.75 wt.-%, preferably 0.005 to 0.60 wt.-% and particularly preferably 0.005 to 0.45 wt.-% peroxide, preferably benzoyl peroxide,
(c) 0.001 to 5.0 wt.-%, preferably 0.003 to 4.0 wt.-%, particularly preferably 0.005 to 3.0 wt.-% thiourea derivative,
(d) 5 to 95 wt.-%, preferably 10 to 95 wt.-% and particularly preferably 10 to 90 wt.-% radically polymerizable monomer,
(e) 0 to 85 wt.-% filler, and
(f) 0.01 to 5 wt.-%, preferably 0.1 to 3 wt.-% and particularly preferably 0.1 to 2 wt.-% additive,
in each case relative to the total mass of the material.

13. Dental material according to claim 12, which comprises 50 to 85 wt.-% or 10 to 70 wt.-% filler.

14. Dental material according to any one of the preceding claims for therapeutic application, preferably as dental cement, filling composite or veneering material.

15. Non-therapeutic use of a dental material according to any one of claims 1 to 13 for the production or repair of dental restorations, such as prostheses, artificial teeth, inlays, onlays, crowns, bridges and complete dentures.

## Revendications

1. Matériau dentaire polymérisable par voie radicalaire, qui contient au moins un monomère polymérisable par voie radicalaire et, en tant que système amorceur pour la polymérisation radicalaire, une association d'un dérivé de thiourée et d'un hydroperoxyde, et ne contient pas d'amine, **caractérisé en ce qu'il** contient en outre au moins un peroxyde en une quantité de 1 à 15 % en poids par rapport à la masse de l'hydroperoxyde, les hydroperoxydes n'étant pas des peroxydes dans le sens de la revendication.

2. Matériau dentaire selon la revendication 1, qui contient en tant qu'hydroperoxyde un composé de formule R-(OOH)ₙ, dans laquelle R est un radical hydrocarboné aliphatique ou aromatique et n vaut 1 ou 2.

3. Matériau dentaire selon la revendication 2, qui contient en tant qu'hydroperoxyde de l'hydroperoxyde de tert-amyle, de l'hydroperoxyde de 1,1,3,3-tétraméthylbutyle, de l'hydroperoxyde de tert-butyle, de l'hydroperoxyde de tert-hexyle, du 2,5-diméthyl-2,5-di(hydroperoxy)hexane, du monohydroperoxyde de diisopropylbenzène, de l'hydroperoxyde de paramenthane, de l'hydroperoxyde de p-isopropylcumène ou un mélange de ceux-ci, de préférence de l'hydroperoxyde de cumène (CHP).

4. Matériau dentaire selon l'une quelconque des revendications précédentes, qui contient en tant que peroxyde de l'α,α-bis(tert-butylperoxy)diisopropylbenzène, du peroxyde de dicumène, du 2,5-diméthyl-2,5-bis(tert-butylperoxy)hexane, du peroxyde de tert-butyle et de cumyle, du peroxyde de di-tert-butyle, du 2,5-diméthyl-2,5-bis(tert-butylperoxy)hexyne-3 ou un mélange de tels peroxydes.

5. Matériau dentaire selon l'une quelconque des revendications précédentes, qui contient en tant que peroxyde un peroxyde de diacyle, de préférence du peroxyde d'isobutyryle, du peroxyde de 2,4-dichlorobenzoyle, du peroxyde de 3,5,5-triméthylhexanoyle, du peroxyde d'octanoyle, du peroxyde de lauroyle, du peroxyde de stéaryle, du peroxyde d'acide succinique, du peroxyde de m-toluoyl-benzoyle ou un mélange de tels peroxydes, de préférence du peroxyde de benzoyle (DBPO).

6. Matériau dentaire selon l'une quelconque des revendications précédentes, qui contient, en tant que dérivé de thiourée, de l'acétyl-, allyl-, pyridyl-, phénylthiourée, hexanoylurée ou un mélange de tels dérivés, de préférence de l'acétylthiourée (ATU).

7. Matériau dentaire selon l'une quelconque des revendications précédentes, qui contient
- 0,01 à 5,0 % en poids, de préférence 0,05 à 4,0 % en poids et de façon particulièrement préférée 0,1 à 3,0 % en poids d'hydroperoxyde, par rapport à la masse totale du matériau,
- 25 à 100 % en moles, de préférence 50 à 100 % en moles et de façon particulièrement préférée une quantité équimolaire de dérivé de thiourée, par rapport à la quantité molaire d'hydroperoxyde,
- 1 à 15 % en poids, de préférence 1 à 10 % en poids et de façon particulièrement préférée 2 à 8 % en poids de peroxyde, par rapport à la masse de l'hydroperoxyde,

8. Matériau dentaire selon l'une quelconque des revendications précédentes, qui contient en tant que monomère polymérisable par voie radicalaire au moins un (méth)acrylate mono- ou multifonctionnel, de préférence au moins un diméthacrylate ou un mélange de mono- et diméthacrylates.

9. Matériau dentaire selon la revendication 8, qui contient en tant que monomère polymérisable par voie radicalaire du (méth)acrylate de méthyle, éthyle, 2-hydroxyéthyle, butyle, benzyle, tétrahydrofurfuryle ou isobornyle, du phénoxy-éthylèneglycolméthacrylate de p-cumyle, du méthacrylate de 2-(2-biphényloxy)-éthyle, du diméthacrylate de bisphénol-A, du bis-GMA, du diméthacrylate de bisphénol-A éthoxylé ou propoxylé, du 2-[4-(2-méthacryloyloxyéthoxyéthoxy)-phényl]-2-[4-(2-méthacryloyloxyéthoxy)phényl]propane), du 2,2-bis[4-(2-méthacryl-oxypropoxy)phényl]propane, un produit d'addition de méthacrylate de 2-hydroxyéthyle et de 1,6-diisocyanate de 2,2,4-triméthylhexaméthylène, un produit d'addition d'un mélange de 0,7 mole de méthacrylate de 2-hydroxyéthyle et 0,3 mole de méthacrylate de 2-hydroxypropyle avec 1 mole de diisocyanate d'α,α,α',α'-tétraméthyl-m-xylylène), du diméthacrylate de di-, tri- ou tétraéthylèneglycol, du triméthacrylate de triméthylolpropane, du tétraméthacrylate de pentaérythritol, du di-et du triméthacrylate de glycérol, du diméthacrylate de 1,4-butanediol, du diméthacrylate de 1,10-décanediol, du bis(méthacryloyloxyméthyl)tricyclo-[5.2.1.02,6]décane, un diméthacrylate de polyéthylèneglycol ou polypropylèneglycol, du diméthacrylate de polyéthylèneglycol-200 ou polyéthylèneglycol-400, du diméthacrylate de 1,12-dodécanediol ou un mélange de ceux-ci.

10. Matériau dentaire selon la revendication 8 ou 9, qui contient en outre au moins un monomère polymérisable par voie radicalaire contenant des groupes acides, de préférence un acide carboxylique, acide phosphorique, apte à la polymérisation, un ester d'acide phosphorique apte à la polymérisation ou un anhydride de ces substances.

11. Matériau dentaire selon l'une quelconque des revendications précédentes, qui contient en outre au moins une charge organique ou inorganique, de préférence un oxyde, tel que SiO₂, ZrO₂ et TiO₂ ou un oxyde mixte à base de SiO₂, ZrO₂, ZnO et/ou TiO₂, une charge microfine ou nanoparticulaire, telle que la silice pyrogénée ou la silice précipitée, de la poudre de verre, telle que de la poudre de quartz, de la poudre de vitrocéramique ou de la poudre de verre opaque aux rayons X, de préférence de la poudre de verre d'aluminosilicate de baryum ou strontium, une charge opaque aux rayons X, telle que le trifluorure d'ytterbium, l'oxyde de tantale(V), le sulfate de baryum, un oxyde mixte de SiO₂ avec l'oxyde d'ytterbium(III) ou l'oxyde de tantale(V), un prépolymère broyé ou un polymère en perles.

12. Matériau dentaire selon l'une quelconque des revendications précédentes, qui contient
(a) 0,01 à 5 % en poids, de préférence 0,05 à 4,0 % en poids et de façon particulièrement préférée 0,1 à 3,0 % en poids d'hydroperoxyde, de préférence d'hydroxyde de cumène,
(b) 0,001 à 0,75 % en poids, de préférence 0,005 à 0,60 % en poids et de façon particulièrement préférée 0,005 à 0,45 % en poids de peroxyde, de préférence de peroxyde de benzoyle,
(c) 0,001 à 5,0 % en poids, de préférence 0,003 à 4,0 % en poids, de façon particulièrement préférée 0,005 à 3,0 % en poids de dérivé de thiourée,
(d) 5 à 95 % en poids, de préférence 10 à 95 % en poids et de façon particulièrement préférée 10 à 90 % en poids de monomère polymérisable par voie radicalaire,
(e) 0 à 85 % en poids de charge, et
(f) 0,01 à 5 % en poids, de préférence 0,1 à 3 % en poids et de façon particulièrement préférée 0,1 à 2 % en poids d'additif,
chaque fois par rapport à la masse totale du matériau.

13. Matériau dentaire selon la revendication 12, qui contient 50 à 85 % en poids ou 10 à 70 % en poids de charge.

14. Matériau dentaire selon l'une quelconque des revendications précédentes, destiné à l'utilisation thérapeutique, de préférence en tant que ciment dentaire, composite d'obturation ou matériau de placage.

15. Utilisation non thérapeutique d'un matériau dentaire selon l'une quelconque des revendications 1 à 13, pour la fabrication ou la réparation de restaurations dentaires, telles que prothèses, dents artificielles, inlays, onlays, couronnes, bridges et prothèses complètes.
